Europäisches Patentamt

European Patent Office

Office européen des brevets

(19)

(11) Veröffentlichungsnummer: **0 401 642**
**A1**

(12) **EUROPÄISCHE PATENTANMELDUNG**

(21) Anmeldenummer: **90110111.3**

(22) Anmeldetag: **28.05.90**

(51) Int. Cl.⁵: **C07C 305/06, C07C 305/10, C11D 1/12**

(30) Priorität: **05.06.89 DE 3918252**

(43) Veröffentlichungstag der Anmeldung:
**12.12.90 Patentblatt 90/50**

(84) Benannte Vertragsstaaten:
**GR**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Fabry, Bernd, Dr.**
**Danzinger Strasse 31**
**D-4052 Korschenbroich 1(DE)**
Erfinder: **Giesen, Brigitte**
**Boeklinstrasse 2**
**D-4000 Düsseldorf(DE)**
Erfinder: **Westfechtel, Alfred, Dr.**
**Kerschensteiner Weg 9**
**D-4010 Hilden(DE)**

(54) **Fettalkylsulfate und Fettalkyl-polyalkylenglycolethersulfate, Verfahren zu ihrer Herstellung und ihre Verwendung.**

(57) Durch Sulfatierung von Isostearylakohol bzw. Isostearyl-poly alkylenglycolethern und anschließende Neutralisation mit geeigneten Basen, werden Alkylsulfate bzw. Ethersulfate mit guten Fließ- und Netzeigenschaften erhalten.

EP 0 401 642 A1

## Fettalkylsulfate und Fettalkyl-polyalkylenglycolethersulfate, Verfahren zu ihrer Herstellung und ihre Verwendung

Die Erfindung betrifft Isostearylalkohol- und Isotstearyl-polyalkylenglycolethersulfate, ein Verfahren zu ihrer Herstellung sowie ihre Verwendung als oberflächenaktive Mittel.

Fettalkylsulfate und Fettalkyl-polyalkylenglykolethersulfate, die im allgemeinen Sprachgebrauch kurz als Alkylsulfate bzw. Ethersulfate bezeichnet werden, sind durch Sulfatierung von Fettalkoholen bzw. durch Sulfatierung von Anlagerungsprodukten von Ethylenoxid und/oder Propylenoxid an Fettalkohole sowie nachfolgender Neutralisation der gebildeten Schwefelsäurehalbester mit geeigneten Basen, z.B. Natronlauge, zugänglich. Als geeignete Sulfatierungsmittel kommen dabei z.B. Schwefelsäure, Oleum, Amidosulfonsäure, insbesondere aber Chlorsulfonsäure oder gasförmiges Schwefeltrioxid in Betracht [J.Am.Oil.Chem. Soc. 36(1959)208-210].

Alkylsulfate mit 16 bis 22 Kohlenstoffatomen sowie Ethersulfate mit Alkylresten gleicher Kettenlänge und eines durchschnittlichen Ethoxylierungsgrades von 1-5 zeigen gute Wascheigenschaften und eine geringe Schaumentwicklung, Eigenschaften, die für den Einsatz von anionaktiven Tensiden z.B. in Universalwaschmitteln vorteilhaft sind. Nachteilig hingegen ist, daß sowohl Alkylsulfate als auch Ethersulfate über ein unzureichendes Netzvermögen verfügen, was ihre Verwendung deutlich einschränkt.

Alkylsulfate und Ethersulfate fallen nach Neutralisation der entsprechenden Schwefelsäurehalbester in Form wäßriger Pasten an. Für die wirtschaftliche Herstellung von pulverförmigen Wasch- und Reinigungsmitteln durch Versprühen sind möglichst hohe Aktivsubstanzkonzentrationen und nur geringe Wassermengen erwünscht. Das gleiche gilt, wenn diese Konzentrate über größere Entfernungen transportiert werden sollen. Die Herstellung von Alkylsulfaten und Ethersulfaten mit mehr als 30 Gew.-% Feststoffgehalt wird jedoch durch die hohe Viskosität der Pasten beeinträchtigt, die durch die Bildung flüssigkristalliner Phasen hervorgerufen wird. Erfahrungsgemäß sind Alkylsulfat- bzw. Ethersulfatpasten mit einem Feststoffgehalt von mehr als 70 Gew.-% nicht mehr pumpbar.

Aufgabe der Erfindung war es daher, Fettalkylsulfate bzw. Fettalkylpolyalkylenglycolethersulfate aufzufinden, die sich gegenüber herkömmlichen Produkten durch ein verbessertes Netzvermögen sowie verbesserte Fließeigenschaften ihrer hochkonzentrierten Lösungen auszeichnen sollten.

Die Fließeigenschaften anionischer Tenside können in der Regel durch den Zusatz von Viskositätsminderern verbessert werden. So ist z.B. aus der EP-B1-24 711 bekannt, daß sich der Zusatz von sulfatierten Polyalkylenglycolen vorteilhaft auf die Viskosität von Aniontensiden auswirkt. Gemäß der Lehre der DE-AS-16 17 160 können zur Senkung der Viskosität auch typische Hydrotrope wie z.B. Cumolsulfonat oder Phosphorsäureester eingesetzt werden.

Überraschenderweise wurde gefunden, daß man aus Isostearylalkohol bzw. Isostearyl-polyalkylenglycolethern Alkylsulfate bzw. Ethersulfate mit so guten Fließeigenschaften herstellen kann, daß sich der bei den herkömmlichen Alkylsulfaten bzw. Ethersulfaten erforderliche Zusatz von Viskositätsminderern erübrigt.

Die Erfindung beruht ferner auf der Erkenntnis, daß sich Isostearylsulfat und Isostearylethersulfate gegenüber vergleichbaren linearen Alkyl- bzw. Ethersulfaten durch ein deutlich besseres Netzvermögen auszeichnen.

Gegenstand der Erfindung sind daher Sulfate von Isostearylalkohol bzw. Isostearyl-polyalkylenglycolethern, die durch Sulfatierung von Isostearylalkohol bzw. Isostearyl-polyalkylenglycolethern der allgemeinen Formel (I),

R-O-[$C_nH_{2n}O$]$_x$H    (I)

in der

n für ganze Zahlen von 2 bis 4

x für 0 oder Zahlen von 0,1 bis 20 und

R für einen methylverzweigten Alkylrest mit insgesamt 18 C-Atomen steht

sowie anschließender Neutralisation der Sulfatierungsprodukte mit Basen erhältlich sind.

Die Ausgangsmaterialien für die Herstellung der erfindungsgemäßen Verbindungen, Isostearylalkohol und Isostearylalkohol-polyalkylenglycolether, stellen bekannte Substanzen dar, die nach üblichen Methoden der organischen Chemie erhalten werden können. Zur Herstellung des Isostearylalkohols [Cosm.Perf. 89,7-(1974)45-47 und Seifen-Öle-Fette-Wachse 114,7(1988)231-233] geht man zweckmäßigerweise von Isostearinsäure aus, die man ihrerseits nach Härtung und Umnetztrennung aus der $C_{18}$-Monomerfraktion der Dimerisierung z.B. von Tallölfettsäure gewinnt. Durch Umsetzung mit Methanol wird die Isostearinsäure in den Methylester überführt und anschließend in an sich bekannter Weise in Gegenwart herkömmlicher Cu/Zn-Katalysatoren bei 200-250°C und 200-300 bar zum Isostearylalkohol hydriert. Der auf diese Weise gewonnene technische Isostearylalkohol enthält 95 bis 100 Gew.-% methylverzweigte Stearylalkohole, bei denen die Methylverzweigungen statistisch um einen Schwerpunkt in 9-Stellung verteilt sind. Darüberhinaus können in Abhängigkeit von der verwendeten Isostearinsäure noch 0 bis 5 Gew.-% an Terpenkohlenwasserstoffen vorhanden sein.

Die Alkoxylierung des Isostearylalkohols kann in an sich bekannter Weise in Gegenwart von 0,1 bis 2 Gew.-% eines basischen Katalysators aus der Gruppe der Alkali- und Erdalkalisalze bei einem Druck von 1-10 bar und einer Temperatur von 120-200° C durchgeführt werden [Falbe u. Hasserodt, Katalysatoren, Tenside u. Mineralöladditive, Thieme-Verlag, Stuttgart, 1978, S. 142]. Durch Anlagerung der erforderlichen Menge Alkylenoxid, z.B. Ethylenoxid und/oder Propylenoxid, die bei gemischten Alkylenoxiden als Random- oder Blockpolymerisation erfolgen kann, werden Isostearyl-polyalkylenglycolether erhalten, die der allgemeinen Formel (I) folgen.

Zur Sulfatierung des Isostearylalkohols sowie der Isostearylpolyalkylenglycolether der allgemeinen Formel (I) können die für die Sulfatierung von Alkoholen üblichen Sulfieragenzien eingesetzt werden, z.B. Schwefelsäure, Oleum oder Amidosulfonsäure, insbesondere aber Chlorsulfonsäure oder gasförmiges Schwefeltrioxid im Gemisch mit einem Inertgas. Die Sulfatierung mit gasförmigem Schwefeltrioxid kann in der für Fettsäure-niedrigalkylester bekannten Weise in hierfür geeigneten Reaktoren, z.B. Fallfilmreaktoren erfolgen. Dabei wird das Schwefeltrioxid mit Luft oder Stickstoff verdünnt und vorzugsweise in Form eines Gasgemisches mit ca. 1 bis 8, insbesondere 3 bis 5 Vol.-% Schwefeltrioxid eingesetzt.

Bei der Sulfatierung können gegebenenfalls sämtliche für die Sulfonierung ungesättigter Fettsäureester, Olefine und Aromaten üblichen inerten Lösungsmittel, wie z.B. Dimethylformamid, 1,2-Dichlorethan oder Orthoameisensäureester eingesetzt werden; bevorzugt wird die Reaktion in Abwesenheit von Lösungsmitteln durchgeführt.

In einer bevorzugten Ausführungsform der Erfindung erfolgt die Herstellung der Verbindungen der allgemeinen Formel (I) durch Umsetzung der Ausgangsprodukte mit Chlorsulfonsäure oder gasförmigem Schwefeltrioxid.

Die Sulfatierung wird zweckmäßigerweise mit einem molaren Verhältnis von Ausgangsprodukt zu Sulfatierungsmittel von 1 : 0,9 bis 1,3, bevorzugt 1 : 1,0 bis 1 : 1,2, bei Temperaturen von 20 bis 80° C, bevorzugt 25 bis 50° C, durchgeführt.

Die bei der Sulfatierung entstehenden sauren Sulfierprodukte, die sich ganz oder überwiegend aus Schwefelsäurehalbestern der Ausgangsprodukte zusammensetzen, werden in wässrige Basen eingerührt und neutralisiert, wobei die Temperatur infolge freiwerdender Neutralisationswärme auf 50 bis 80° C ansteigen kann.

Als Basen für die Neutralisation kommen Alkalimetallhydroxide wie Natrium-, Kalium- und Lithiumhydroxid, Erdalkalimetalloxide und -hydroxide wie Magnesiumoxid, Magnesiumhydroxid, Calciumoxid und Calciumhydroxid, Ammoniak, Mono-, Di- und Tri-$C_2$- bis $C_4$-alkanolamine, beispielsweise Mono-, Di- und Triethanolamin sowie primäre, sekundäre oder tertiäre $C_1$- bis $C_4$-Alkylamine in Betracht. Die Neutralisationsbasen kommen dabei vorzugsweise in Form mehr oder weniger konzentrierter wäßriger Lösungen zum Einsatz, wobei 25 bis 55 gew.-%ige Natriumhydroxidlösungen bevorzugt sind.

In einer bevorzugten Ausführungsform der Erfindung werden in den aus dem Verfahren resultierenden wässrigen Lösungen der Isostearylalkohol-bzw. Isostearyl-polyalkylenglycolethersulfate Feststoffgehalte von 30 bis 70 Gew.-% eingestellt. Zu diesem Zweck werden die sauren Sulfierprodukte in wässrige Lösungen der Neutralisationsbasen eingerührt, die nur soviel Wasser enthalten, wie für die Einstellung des gewünschten Feststoffgehaltes im Endprodukt erforderlich ist.

Falls dies gewünscht wird, können die erfindungsgemäßen Sulfate in an sich bekannter Weise durch Zusatz von Wasserstoffperoxid- oder Natriumhypochloritlösung gebleicht werden. Dabei werden, bezogen auf den Feststoffgehalt in der Lösung der Sulfate, 0,2 bis 2 Gew.-% Wasserstoffperoxid, berechnet als 100 %ige Substanz, oder entsprechende Mengen Natriumhypochlorit eingesetzt.

Die erfindungsgemäß hergestellten Sulfate des Isostearylalkohols sowie der Isostearyl-polyoxyalkylether können durch die Formel (II) beschrieben werden,

R-O-[$C_nH_{2n}O]_x$SO$_3$X     (II)

in der
n für ganze Zahlen von 2 bis 4,
x für 0 oder Zahlen von 0,1 bis 20
R für einen gesättigten methylverzweigten Alkylrest mit insgesamt 18 C-Atomen und
X für Wasserstoff, ein Alkali- oder Erdalkalimetall, einen Ammonium- oder Aminrest steht.

Die Erfindung betrifft weiterhin die Verwendung der durch Sulfatierung von Isostearylalkohol bzw. Isostearyl-polyalkylenglycolethern erhältlichen bzw. hergestellten Sulfate von Isostearylalkohol bzw. Isostearyl-polyalkylenglycolethern als oberflächenaktive Mittel, insbesondere in Universalwaschmitteln.

3

EP 0 401 642 A1

BEISPIELE

Herstellung der erfindungsgemäßen Sulfate

Beispiel 1: Sulfatierung von Isostearylalkohol.

In einem 1-l-Sulfierreaktor mit Mantelkühlung wurden 281 g (1 Mol) technischer Isostearylalkohol (OHZ = 200, Fa.Henkel) vorgelegt und bei 25 °C mit 84 g (1,05 Mol) gasförmigem Schwefeltrioxid umgesetzt. Das Schwefeltrioxid wurde durch Erhitzen aus einer entsprechenden Menge 65 %-igen Oleums ausgetrieben, mit Stickstoff auf eine Konzentration von 5 Vol.-% verdünnt und innerhalb von 35 min in den Alkohol eingeleitet. Die $SO_3$-Aufnahme (gravimetrisch bestimmt) betrug 98 %. Nach der Sulfatierung wurde das saure Reaktionsgemisch in wäßrige 25 %ige Natronlauge eingerührt und neutralisiert.
Kenndaten des Produktes:
Aniontensidgehalt = 25,0 Gew.-% = 0,065 mEq/g
Unsulfierte Anteile = 2,6 Gew.-%
Sulfat (berechnet als $Na_2SO_4$) = 1,4 Gew.-%
Wasser (nach Fischer) = 71,0 Gew.-%

Beispiel 2: Sulfatierung von Isostearylalkohol-2EO.

Beispiel 1 wurde unter Verwendung von 369 g (1 Mol) eines Anlagerungsproduktes von durchschnittlich 2 Mol Ethylenoxid an 1 Mol technischen Isostearylalkohol (OHZ = 152) gemäß Beispiel 1 wiederholt. Die Umsetzung mit 1 Mol Schwefeltrioxid erfolgte bei 50 °C.
Kenndaten des Produktes:
Aniontensidgehalt = 15,2 Gew.-% = 0,032 mEq/g
Unsulfierte Anteile = 4,1 Gew.-%
Sulfat (berechnet als $Na_2SO_4$) = 1,2 Gew.-%
Wasser (nach Fischer) = 79,5 Gew.-%

Vergleichsbeispiel A: Sulfatierung von Talgalkohol.

Beispiel 1 wurde unter Verwendung von 258 g (1 Mol) technischen Talgalkohols (OHZ = 217, Stenol[R] 1618 der Fa.Henkel) wiederholt. Die Umsetzung mit 1 Mol Schwefeltrioxid erfolgte bei 50 °C.
Kenndaten des Produktes:
Aniontensidgehalt = 26,1 Gew.-% = 0,072 mEq/g
Unsulfierte Anteile = 1,8 Gew.-%
Sulfat (berechnet als $Na_2SO_4$) = 1,4 Gew.-%
Wasser (nach Fischer) = 70,7 Gew.-%

Vergleichsbeispiel B: Sulfatierung von Talgalkohol-2EO.

Beispiel 1 wurde unter Verwendung von 346 g (1 Mol) eines Anlagerungsproduktes von durchschnittlich 2 Mol Ethylenoxid an 1 Mol technischen Talgalkohol (OHZ = 162) gemäß Vergleichsbeispiel A wiederholt. Die Umsetzung mit 1 Mol Schwefeltrioxid erfolgte bei 35 °C.
Kenndaten des Produktes:
Aniontensidgehalt = 17,1 Gew.-% = 0,038 mEq/g
Unsulfierte Anteile = 2,3 Gew.-%
Sulfat (berechnet als $Na_2SO_4$) = 1,2 Gew.-%
Wasser (nach Fischer) = 79,4 Gew.-%
Der Aniontensid-Gehalt (WAS) und die unsulfierten Anteile (US) wurden nach den DGF-Einheitsmethoden, Stuttgart 1950 - 1984, H-III-10 bzw. G-II-6b ermittelt.

4

Bestimmung der Pastenviskosität

Die Bestimmung der Pastenviskosität erfolgte in einem Brookfield LVT-Viskosimeter bei 50 und 90°C, Spindel 4 und einer Rotation von 60 U.min-1. Zur Herstellung der 30 bzw. 70 gew.-%igen Pasten wurden die Produkte gemäß den Beispielen 1 und 2 sowie den Vergleichsbeispielen A und B am Rotationsverdampfer bis auf den entsprechenden Gehalt an Aktivsubstanz entwässert. Die Ergebnisse sind in Tabelle 1 zusammengefaßt.

Tabelle 1:

| Pastenviskositäten | | | | |
|---|---|---|---|---|
| Beispiel | 30 gew.-%ige Pasten | | 70 gew.-%ige Pasten | |
| | 50°C | 90°C | 50°C | 90°C |
| | mPa.s | mPa.s | mPa.s | mPa.s |
| 1 | 3920 | 3480 | 7820 | 6940 |
| 2 | 6500 | 6180 | 11400 | 10050 |
| A | 6910 | 4850 | 15600 | 13850 |
| B | 8550 | 7920 | 19750 | 17300 |

Man erkennt, daß die erfindungsgemäßen Isostearylsulfate und Isostearylethersulfate sowohl bei niedrigen als auch bei hohen Pastenkonzentrationen gegenüber den Vergleichsbeispielen deutlich niedrigere Viskositäten aufweisen.

Bestimmung des Netzvermögens

Das Netzvermögen der Produkte wurde gemäß DIN 53901 nach der Tauchnetzmethode in weichem (0°d) und hartem (16°d) Wasser mit 1 g AS/l bestimmt. Die Ergebnisse sind in Tabelle 2 zusammengefaßt.

Tabelle 2:

| Tauchnetzvermögen | | | |
|---|---|---|---|
| Beispiel | Konsistenz | $t_n(0°d)$ | $t_n(16°d)$ |
| | 10 gew.-%ige Lösung | s | s |
| 1 | flüssig | 132 | 318 |
| 2 | flüssig | 208 | 388 |
| A | fest | > 400 | > 400 |
| B | fest | > 400 | > 400 |

Man erkennt, daß die erfindungsgemäßen Isostearylsulfate bzw. Isostearylethersulfate gegenüber den Vergleichsbeispielen ein besseres Netzvermögen aufweisen.

**Ansprüche**

1. Sulfate von Isostearylalkohol bzw. Isostearyl-polyalkylen glycolethern, erhältlich durch Sulfatierung von Isostearylalkohol bzw. Isostearyl-polyalkylenglycolethern der allgemeinen Formel (I)

$$R-O-[C_nH_{2n}O]_xH \quad (I)$$

in der

n für ganze Zahlen von 2 bis 4 x für 0 oder Zahlen von 0,1 bis 20 und R für einen gesättigten verzweigten Alkylrest mit insgesamt 18 C-Atomen steht
sowie anschließender Neutralisation der Sulfatierungsprodukte mit Basen.

2. Sulfate von Isostearyl-polyalkylenglycolethern nach Anspruch 1, erhältlich durch Sulfatierung von Isostearyl-polyoxyalkylethern der allgemeinen Formel (I), in der
n für die Zahl 2 und x für Zahlen von 0 bis 5 steht.

3. Sulfate von Isostearylalkohol bzw. Isostearyl-polyalkylen glycolethern nach mindestens einem der Ansprüche 1 und 2, dadurch gekennzeichnet, daß man die Sulfatierung mit Chlorsulfonsäure oder gasförmigem Schwefeltrioxid durchführt.

4. Sulfate von Isostearylalkohol bzw. Isostearyl-polyalkylenglycolethern nach mindestens einem der Ansprüche 1 bis 3, dadurch gekennzeichnet, daß man die Sulfatierung mit einem molaren Verhältnis Ausgangsprodukt : Sulfatierungsmittel von 1 : 0,9 bis 1 : 1,3, insbesondere 1 : 1,0 bis 1 : 1,2 gegebenenfalls in inerten Lösungsmitteln durchführt.

5. Sulfate von Isostearylalkohol bzw. Isostearyl-polyalkylenglycolethern nach mindestens einem der Ansprüche 1 bis 4, dadurch gekennzeichnet, daß man die Sulfatierung bei Temperaturen von 20 bis 80° C, insbesondere 25 bis 50° C durchführt.

6. Sulfate von Isostearylalkohol bzw. Isostearyl-polyalkylenglycolethern nach mindestens einem der Ansprüche 1 bis 5, dadurch gekennzeichnet, daß man die Sulfatierung mit Schwefeltrioxid durchführt.

7. Sulfate von Isostearylalkohol bzw. Isostearyl-polyalkylen glycolethern nach mindestens einem der Ansprüche 1 bis 6, dadurch gekennzeichnet, daß man die Neutralisation mit wässrigen Basen aus der von Alkalimetallhydroxiden, Erdalkalimetalloxiden und -hydroxiden, Ammoniak, Mono-, Di- und Tri-C$_2$- bis -C$_4$-Alkanolaminen sowie primären, sekundären und tertiären C$_1$- bis C$_4$-Alkylaminen gebildeten Gruppe durchführt.

8. Wässrige Lösungen von Sulfaten von Isostearylalkohol bzw. Isostearyl-polyalkylenglycolethern nach mindestens einem der Ansprüche 1 bis 7 mit einem Feststoffanteil von 30 bis 70 Gew.-%.

9. Verfahren zur Herstellung von Sulfaten von Isostearylalkohol bzw. Isostearyl-polyalkylenglycolethern, dadurch gekennzeichnet, daß man Isostearylalkohol bzw. Iso stearyl-polyalkylenglycolether der allgemeinen Formel (I),

R-O-[C$_n$H$_{2n}$O]$_x$H    (I)

in der
n für ganze Zahlen von 2 bis 4 x für 0 oder Zahlen von 0,1 bis 20 und R für einen methylverzweigten Alkylrest mit insgesamt 18 C-Atomen steht,
sulfatiert und anschließend mit Basen neutralisiert.

10. Verfahren nach Anspruch 9, dadurch gekennzeichnet, daß man Isostearyl-polyalkylenglycolether der allgemeinen Formel (I), in der
n für die Zahl 2 und
x für Zahlen von 0,1 bis 5 steht,
sulfatiert.

11. Verfahren nach Anspruch 9 oder 10, dadurch gekennzeichnet, daß man die Sulfatierung mit Chlorsulfonsäure oder Schwefeltrioxid, gegebenenfalls in einem inerten Lösungsmittel durchführt.

12. Verfahren nach mindestens einem der Ansprüche 9 bis 11, dadurch gekennzeichnet, daß man die Sulfatierung mit einem molaren Verhältnis Ausgangsprodukt:Sulfatierungsmittel von 1 : 0,9 bis 1 : 1,3, insbesondere 1 : 1,0 bis 1 : 1,2 durchführt.

13. Verfahren nach mindestens einem der Ansprüche 9 bis 12, dadurch gekennzeichnet, daß man die Sulfatierung bei Temperaturen von 20 bis 80° C, bevorzugt 25 bis 50° C durchführt.

14. Verfahren nach mindestens einem der Ansprüche 9 bis 13, dadurch gekennzeichnet, daß man die Neutralisation mit wässrigen Basen aus der von Alkalimetallhydroxiden, Erdalkalimetalloxiden und -hydroxiden, Ammoniak, Mono-, Di- und Tri-C$_2$- bis -C$_4$-alkanolaminen sowie primären, sekundären und tertiären C$_1$- bis C$_4$-Alkylaminen gebildeten Gruppe durchführt.

15. Verfahren nach mindestens einem der Ansprüche 9 bis 14, dadurch gekennzeichnet, daß man in den wässrigen Lösungen der Isostearylalkohol- bzw. Isostearyl-polyalkylenglycol ethersulfate Feststoffgehalte von 30 bis 70 Gew.-% einstellt.

16. Verwendung der Sulfate von Isostearylalkohol bzw. Isostearylpolyalkylenglycolether nach mindestens einem der Ansprüche 1 bis 8 als oberflächenaktive Mittel, insbesondere in Universalwaschmitteln.

17. Verwendung der nach dem Verfahren nach mindestens einem der Ansprüche 9 bis 15 erhältlichen bzw. hergestellten Sulfate von Isostearylalkohol bzw. Isostearyl-polyalkylenglycolethern als oberflächenaktive Mittel, insbesondere in Universalwaschmitteln.

6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | AT-B- 353 761 (UNILEVER) <br> * Patentanspruch * <br> --- | 1-7,9-14 | C 07 C 305/06 <br> C 07 C 305/10 <br> C 07 C 303/24 <br> C 11 D 1/14 <br> C 11 D 1/29 |
| X | US-A-3 338 949 (H.J. HAGEMEYER, Jr. et al.) <br> * Spalte 1, Zeilen 13-10; Beispiele 1,4; Ansprüche * <br> --- | 1,7,9, 16-17 | |
| X | US-A-3 376 333 (R. ERNST et al.) <br> * Anspruch 1 * <br> --- | 1-2,10 | |
| A | DE-A-1 618 524 (IMPERIAL CHEMICAL INDUSTRIES) <br> * Ansprüche 1,8,9 * <br> --- | 1,3,6-7,9,11, 16-17 | |
| A | US-A-3 647 851 (J.B. WILKES) <br> * Anspruch 1 * & DE-B-1 768 760 <br> --- | 1,3-5,7,9,11-14 | |
| A | US-A-2 654 772 (A.A. PAULIC) <br> * Ansprüche * <br> --- | 1,7 | RECHERCHIERTE SACHGEBIETE (Int. Cl.5) |
| A | US-A-2 644 831 (M. KOSMIN) <br> * Anspruch 1; Spalte 1, Zeilen 1-4 * <br> --- | 1-2,7, 16-17 | C 07 C 305/00 <br> C 07 C 303/00 |
| A,D | EP-A-0 024 711 (HENKEL) <br> * Anspruch 1; Spalten 6-I,7-III * <br> --- | 1,8,16-17 | |
| X | DE-A-2 127 033 (AGFA-GEVAERT) <br> * Seite 5, Herstellung 1 * <br> ----- | 1,3-4,6,9 | |

Der vorliegende Recherchenbericht wurde für alle Patentansprüche erstellt

| Recherchenort | Abschlußdatum der Recherche | Prüfer |
|---|---|---|
| DEN HAAG | 16-07-1990 | VAN GEYT J.J.A. |

EPO FORM 1503 03.82 (P0403)